Europäisches Patentamt

European Patent Office

Office européen des brevets

(11) Veröffentlichungsnummer: **0 205 660**
A2

(12) **EUROPÄISCHE PATENTANMELDUNG**

(21) Anmeldenummer: 85114533.4

(22) Anmeldetag: 15.11.85

(51) Int. Cl.⁴: **A 61 L 15/00**, A 61 L 31/00, A 61 B 19/08, A 41 D 13/12

(30) Priorität: 20.06.85 DE 3522051

(43) Veröffentlichungstag der Anmeldung: 30.12.86 Patentblatt 86/52

(84) Benannte Vertragsstaaten: AT BE CH DE FR GB IT LI LU NL SE

(71) Anmelder: **Firma Carl Freudenberg, Höhnerweg 4, D-6940 Weinheim/Bergstrasse (DE)**

(72) Erfinder: **Groitzsch, Dieter, Dr., Hermann-Löns-Strasse 6A, D-6945 Hirschberg 2 (DE)**
Erfinder: **Keil, Andreas, Oberliebersbach 19, D-6942 Mörlenbach (DE)**

(74) Vertreter: **Weissenfeld-Richters, Helga, Dr., Höhnerweg 2, D-6940 Weinheim/Bergstrasse (DE)**

(54) Wegwerfbares Abdeck- oder Bekleidungstuch für die Chirurgie und Verfahren zu dessen Herstellung.

(57) Es wird ein wegwerfbares Abdeck- oder Bekleidungstuch für die Chirurgie aus Vliesstoff und einem darauf befindlichen, verpreßten Polymerschaumfilm beschrieben, welcher mit Hydrophobiermitteln ausgerüstet ist.
    Weiter wird ein geeignetes Herstellungsverfahren beansprucht.

EP 0 205 660 A2

ACTORUM AG

**Dr. H. Weissenfeld-Richters**
Patentanwältin

Höhnerweg 2-4
6940 Weinheim/Bergstr.
Telefon (0 62 01) 80-8618
Telex 4 65 531

0205660

11. November 1985
Ho/Sch
ON 5036/Europa

1

Anmelderin: Firma Carl Freudenberg, Weinheim

## Wegwerfbares Abdeck- oder Bekleidungstuch für die Chirurgie und Verfahren zu dessen Herstellung

Die Erfindung betrifft ein wegwerfbares Abdeck- oder Bekleidungstuch für die Chirurgie, welches aus einem wasserdampfdurchlässigen, drapierfähigen Trägervliesstoff und einer diesen nicht durchdringenden, polymeren Deckfolie besteht.

Solche wegwerfbaren Tücher werden in zunehmendem Maße
in der Chirurgie eingesetzt, da der einmalige Gebrauch
absolute Hygiene gewährleistet und somit die postoperative Infektionsrate bei den Patienten im Vergleich zu
gewaschenen, wiederverwendbaren OP-Gewebetüchern aus
Baumwolle deutlich gesenkt werden konnte.

Das Anwendungsgebiet der Erfindung liegt also einerseits
in der Schutzkleidung für die am operativen Eingriff beteiligten Personen, zum anderen in der Abdeckung des
Patienten im nicht unmittelbar an die Inzisionsstelle
(Operationswunde) anschließenden Peripheriebereich. Für
beide Anwendungsgebiete werden im wesentlichen folgende
Anforderungen an ein Einwegtuch gestellt:
Hohe Wasserdampfdurchlässigkeit bei hohem Wasseraufnahmevermögen (Atmungsaktivität, Wasserhaushalt),
Wasserdichtheit nach DIN 53 886-77 in der Größenordnung
von mindestens $2 \cdot 10^{-5}$ Pa,
Lint-Freiheit bzw. hohe Abriebbeständigkeit und sehr
hohe Drapierfähigkeit (d.h. textiler "Fall") sowie ausreichende mechanische Festigkeit, um die Kräfte bei
Konfektionierung und praktischem Gebrauch zu überstehen.
Unter Lint-Freiheit versteht man die Eigenschaft eines
Textilmaterials, beim Gebrauch keine Partikeln, wie
Faserbruchstücke bzw. Faserstaub, abzugeben oder freizusetzen. Solche freigesetzten, in der Luft des Operationssaales herumvagabundierenden Partikeln können als Keimträger fungieren und somit zur postoperativen Wundinfektion führen. Insbesondere die wiederverwendbaren,
konventionellen Baumwollgewebe neigen mit steigender Zahl
der Wäschen immer stärker zu Linting.

3

Eine wegwerfbare Einweg-Operationsabdeckung erfüllt ihre
wesentlichste Funktion als Bakteriensperre bzw. Infektionsverhüter um so perfekter, je geringer ihre Lint-Rate und
je dichter sie gegenüber Wasser, Alkohol und physiologischen Flüssigkeiten ist.

Die bekannten Verbundstoffe aus Folie/Vliesstoff bzw.
Folie/Tissue-Papier bzw. Tissue/Folie/Vliesstoff sind
zwar absolut luft- und wasserdicht und somit auch absolut
keimdicht, haben aber den großen Nachteil, daß sie
papierig, steif, untextil und schlecht drapierfähig sind,
einen Raschelgriff aufweisen und zu einem Hitzestau
beim Patienten, insbesondre bei länger andauernden
Operationen, führen.

Bekannt sind weiterhin mit Wasserstrahlen extrem hohen
Druckes vernadelte Verbundstoffe aus Polyesterfasern und
einer Tissue-Lage. Diese sogenannten spunlaced-Produkte
weisen die oben genannten Nachteile nicht auf. Sie können
aufgrund der bindemittelfreien Ausrüstung gute Bakterien-
Sperrfunktion mit hoher Textilität und Drapierfähigkeit
verbinden, haben jedoch den großen Nachteil deutlich
höherer Lint-Raten als bei Naßvliesstoffen, was insbesondere für die relativ offen strukturierte Polyesterseite gilt. Dies ist im wesentlichen darauf zurückzuführen,
daß die verwendeten Cellulose-Fibriden eine geringe Eigenbindung miteinander aufweisen und für eine ausreichende
Faserverschlingung zu kurz sind.

Aufgabe der vorliegenden Erfindung ist es, ein chirurgisches Abdecktuch aus Vliesstoff für den Einmalgebrauch anzugeben, das die genannten Anforderungen erfüllt, die vorstehenden Nachteile bekannter Stoffe überwindet und auch als OP-Bekleidung geeignet ist. Ferner soll ein Verfahren zur Herstellung eines solchen Tuches aufgezeigt werden.

Als Lösung wird ein wegwerfbares Abdeck- oder Bekleidungstuch vorgeschlagen, welches eine Trägervliesstoffschicht und eine diese nicht durchdringende polymere Deckschicht aufweist und die Merkmale des kennzeichnenden Teiles des Anspruches 1 besitzt. Die erfindungsgemäße Herstellung verläuft in der Weise, daß mittels der CRUSHED FOAM-Technik auf einen drapierfähigen Trägervliesstoff einseitig mittels Rakeln und/oder Walzen eine wäßrige Polymerschaumdispersion aufgetragen wird, die auf der Basis Polyacrylester, Polyvinylacetatacrylat oder Nitrilbutadien besteht und emulgatorarm ist, wobei ihr Schaumgewicht weniger als 0,2 g/cm³ und ihr Auftragungsgewicht 15 bis 50 g/m² beträgt und der Schaum - jeweils bezogen auf sein Gesamtgewicht -
1 bis 15 % Hydrophobiermittel,
3 bis 15 % eines Schaumstabilisators, bestehend aus einem Ammonium-oder Alkylammoniumsalz einer langkettigen Fettsäure mit einer Kohlenstoffzahl zwischen 14 und 20
und mindestens 0,25 % Methyl-oder Methylhydroxyalkylcellulose als Verdicker
enthält.

Das Prinzip dieses CRUSHED FOAM-Beschichtungsverfahrens auf der Basis wäßriger Polyacrylsäureester-Dispersionen ist an sich bekannt:
Man mischt der polymeren, dispersen Streichpaste Bindemitteldispersionen, Schaumstabilisatoren, Verdickungsmittel und gegebenenfalls Schaumhilfsmittel und Füllstoffe zu, schäumt das Ganze auf und trägt den Schaum mittels Rakeln und/oder Rollen auf das textile Basismaterial auf. Anschließend wird langsam getrocknet und die Schaumschicht zwischen kalten oder beheizten Walzen verpreßt.

Das obige Verfahren hat sich bisher für die Herstellung weitgehend wasserdichter Einweg-OP-Vliesstoffe als nicht geeignet erwiesen. Wegen der offenen Struktur der Vliesstoff-Basismaterialien sowie des Schaumstoff-Filmes ist die Wasserdichtheit des Produktes unzureichend, auch wenn es nachträglich hydrophobiert wird. Selbst die Zugabe eines Hydrophobiermittels in die aufzuschäumende Streichpaste führt nicht zu brauchbaren OP-Stoffen, da hierdurch die Stabilität des Schaumes drastisch herabgesetzt wird und dieser demzufolge unerwünschtermaßen in das Trägermaterial eindringt. Ferner bekannt sind die genannten, als wäßrige Dispersionen schäumbaren Polymeren. Jedoch erst die erfindungsgemäße, komplette Mischungszusammensetzung der dispersen Streichpaste führte überraschenderweise zu der Möglichkeit, die Schäume mit den für die OP-Anwendung notwendigen Hydrophobierungsmitteln auszurüsten, ohne daß die Stabilität des Schaumes herabgesetzt wird bzw. die Schaummasse in den Trägerstoff eindringt.

Die Viskositäten der ungeschäumten Mischung, gemessen
nach Brookfield (DIN 53 019 für Rotationsviskosimeter),
bewegen sich innerhalb 0,8 und 6 Pa s. Als vorteilhaft haben sich Viskositäten von 1 bis 2 Pa s erwiesen.

Zur Erreichung einer hohen Wasserdichtheit mit möglichst
niedrigem Schaumauftrag und einer hohen Drapierfähigkeit ist es notwendig, mit einem sehr niedrigen Schaumgewicht von weniger als 200 g/l zu arbeiten. Dadurch wird
eine extrem hohe Schaumviskosität bzw. Schaumsteifigkeit erreicht, die ein Eindringen der Beschichtung
während des Schaumauftrages und des anschließenden
Trockenprozesses zuverlässig verhindert.

In den Fällen, wo die Bindemitteldispersionen schlecht
schäumbar sind bzw. nur einen grobporigen Schaum ergeben, ist der Zusatz einer geringen Menge (1 Gew.-%)
eines Schaumhilfsmittels notwendig. Dieser Verfahrensschritt ist jedoch dem Fachmann geläufig und nicht
Gegenstand der vorliegenden Erfindung.

Das Produkt ist allein aufgrund der Eigenschaften der
Schaumschicht ausreichend wasserdicht (mindestens $2 \cdot 10^{-5}$ Pa
nach DIN 53 886-77), ohne daß der Trägervliesstoff
zusätzlich hydrophobiert werden müßte. Die Hydrophobie
des Schichtstoffes läßt sich also allein durch die
Menge der CRUSHED FOAM-Folie für die meisten Anwendungszwecke ausreiched einstellen, wobei Flächengewichte
der Folie von 15 g/m² bei hydrophob ausgerüsteten und
von 50 g/m² bei nicht hydrophoben Trägervliesstoffen
sinnvolle Grenzen darstellen.

Höhere Mengen würden die Drapierfähigkeit des Tuches
zu sehr vermindern.

Erfindungsgemäß gibt man der schäumfähigen Streichpaste
1 bis 15 Gew.-% (Gehalte jeweils auf das Schaumgewicht
bezogen) an Hydrophobiermitteln zu, wobei 2 bis 15 Gew.-%
eines in Wasser nicht löslichen, aber dispergierbaren
Ammonium-oder Alkylammoniumsalzes einer langkettigen
($C_{14}$ bis $C_{20}$) Fettsäure sowie mindestens 0,25 Gew.-%
einer hochmolekularen Methyl- oder Methylhydroxyalkylcellulose als Schaumstabilisator bzw. Verdickungsmittel
beigemischt werden müssen. Unter den Salzen einer langkettigen Fettsäure hat sich Ammoniumstearat als besonders vorteilhaft in der Wirkung erwiesen.

Aufgrund der Tatsache, daß der oberflächenaktive Einfluß von Verdickern mit der Anzahl der Methylgruppen
steigt, sind mittel- bis hochverätherte Methyl- oder
Methylhydroxyalkylcellulosen besonders geeignet.
Carboxymethylcellulosen, Stärke,Alginate, Polyacrylate
und andere natürliche, halb- oder vollsynthetische Verdickungsmittel können nicht angewendet werden.

Als für die erfindungsgemäße Streichpaste bevorzugte
Hydrophobierungsmittel wurden gefunden:
Fettsäuremodifizierte Melaminharze,
deren Mischungen mit Paraffin,
zirkoniumsalzhaltige, wäßrige Paraffinemulsionen,
polymere fluorierte Kohlenwasserstoffe
sowie deren Mischungen mit den obigen Substanzen.

In einer besonderen Ausgestaltungsform der Erfindung kann der weitgehend wasserdicht beschichtete Vliesstoff zusätzlich gewaschen und nachträglich hydrophob ausgerüstet werden. Die damit verbundene Steigerung der Wasserdichtheit ist in diesem Fall jedoch ausschließlich auf ein Auswaschen der Faseravivagen des Trägervliesstoffes und auf die Hydrophobierung des unbeschichteten Fasermaterials und nicht auf eine zusätzliche Verbesserung der Wasserdichtheit innerhalb der Schaumbeschichtung zurückzuführen. Zweckmäßigerweise werden dieser Waschvorgang und die Hydrophobierung ohne Zwischentrocknung, d.h. durch die sogenannte naß-in-naß-Tränkmethode, vorgenommen.

Mit dem beanspruchten Verfahren ist es somit möglich, selbst sehr offen strukturierte, schwach abgebundene, flusige und stark saugfähige Vliesstoffe mit einer geschlossenen Oberflächenbeschichtung zu versehen, die gegen Wasserdurchtritt schützt und gleichzeitig extrem gute Drapierfähigkeit verleiht.

Bei der Verwendung von druckgebundenen, detergenzienhaltigen Vliesstoffen mit hydrophilem Bindersystem ist ein Waschprozeß entweder vor oder nach der Schaumbeschichtung unumgänglich, um wäßrige Präparationen mit grenzflächenaktiven Eigenschaften abzulösen.

Als weiche, drapierfähige Beschichtungsträger bieten sich auch wasserstrahlvernadelte, bindemittelfreie Vliesstoffe mit oder ohne Strukturierung an.

Solche Materialien haben den besonderen Vorteil, daß sie absolut frei von wasserlöslichen Bestandteilen, wie Spinnpräparationen oder sonstigen Hilfsmitteln, sind.

Für chirurgische Schutzbekleidungen kann der Trägervliesstoff im Interesse eines hohen Wasserhaushaltes Baumoder Zellwolle enthalten, um den Tragekomfort zu verbessern.

Das erfindungsgemäße Verfahren ist also für alle bekannten, gut drapierfähigen Trägervliesstoffe anwendbar.

Bei der Verarbeitung von Polymerdispersionen mit einem sehr weichen Film oder gar einem Verschnitt mit Klebstoff-Rohprodukten ist die Klebrigkeit der Beschichtung durch eine Nachbehandlung mit Antiblockmitteln zu beheben. Hierzu zählen beispielsweise Vollbad-Nachtränkungen mit Silikonen und die nur die Schaumoberflächen abdeckende Talkumierung: Talkum wird zweckmäßigerweise vor dem Zusammenpressen der Schaumbeschichtung aufgepudert. Beim Pressen kann es dann in den Schaum oberflächlich eingedrückt werden. Überflüssiger Puder wird abgebürstet oder abgeschüttelt und mittels Vakuum abgesaugt. Talkum ist deswegen besonders vorteilhaft, weil bereits mit geringsten Auftragsmengen hervorragende Antiblockeigenschaften und eine zusätzliche Griffverbesserung (samtiger Griff) erreicht werden können.

Anstelle der Talkumierung können auch Mikrofasern aus hydrophoben Hartpolymeren, wie z.B. Polycarbonaten, durch elektrostatisches Aufspinnen aufgetragen und in den Schaum eingepreßt werden.

In den Fällen, wo eine Saugfähigkeit oder Benetzbarkeit des Basismaterials nicht erwünscht oder notwendig ist, wird zweckmäßigerweise das beschichtete Material gewaschen und hydrophobiert, wobei solche Hydrophobiermittel eingesetzt werden, die gleichzeitig als Antiblockmittel wirken, so z.B. kationenaktive Emulsionen aus fluorierten oder teilfluorierten Kohlenwasserstoffen. Damit können in einem einzigen Schritt gleichzeitig das Trägervlies wasserdicht und die Beschichtung klebfrei ausgerüstet werden.

Die folgenden Beispiele sollen zuerst den Stand der Technik verdeutlichen und danach einige als besonders vorteilhaft gefundene Produkte/Verfahren aufzeigen.

Vergleichsbeispiel 1, entsprechend dem Stand der Technik

Ein quergelegtes Faservlies aus 90 % Perlon-Stapelfasern (dtex 1,7/40 mm, matt) und 10 % Viskose-Zellwolle (dtex 1,3/40 mm, glänzend) wurde in einem Kalander zwischen einer beheizten, glatten Stahlwalze und einer Stahlrasterwalze thermisch verfestigt. Die Rastertiefe betrug 0,51 mm. Beide Walzen wurden auf 211 °C aufgeheizt.

11

Der Kalanderliniendruck betrug 686,5 N/cm. Der Anteil
der verschweißten Faserflächen an der Gesamtvliesstoff-
Fläche betrug 24 %. Dieser bindemittelfreie Trägervliesstoff wurde mit einer handelsüblichen, geschäumten Polyacrylat-Pastenmischung beschichtet. Die Viskosität der ungeschäumten Paste betrug 1,25 Pa s , gemessen mit Spindel
Nr. 3 bei einer Umdrehungszahl von 20 U/min (DIN 53 019,
Brookfield-Rotationsviskosimeter). Die Paste wurde mit
einem Schaummischer auf eine Dichte von 0,20 g/cm³
aufgeschäumt. Ihre Viskosität betrug 7,50 Pa s,
gemessen nach Brookfield mit Spindel Nr. 4 bei 20 U/min.

Auf den 35 g/m² schweren Basisvliesstoff wurden 30 g/m²
Schaumpaste aufgestrichen und in einem Bandktrockner bei
130 °C getrocknet. Der getrocknete Schaum wurde dann
zwischen ungeheizten Stahlwalzen mit einem Liniendruck
von 98 N/cm zusammengedrückt. Anschließend wurde die
beschichtete Ware mit einer 3%igen Hydrophobiermittelmischung
eines paraffinhaltigen, fettsäuremodifizierten Melaminharzes getränkt. Die Naßaufnahme betrug nach dem Abquetschen der Flotte 100 %, bezogen auf das Trockengewicht des Vliesstoffes. Getrocknet und vernetzt wurde
in einem Bandtrockner bei  150 °C.

Das Fertigmaterialgewicht betrug 67 g/m². Die Ware hatte
vor der wasserabweisenden Ausrüstung eine Wasserdichtheit,
gemessen nach DIN 53 886-77, von $6 . 10^{-6}$ Pa und nach
der Ausrüstung einen Wert von $1,7 . 10^{-5}$ Pa.

12

Diese Wasserdichtheit ist für den Einsatz als OP-Abdeckvliesstoff bez. OP-Kittelmaterial nicht ausreichend. Hier
werden Dichtheiten von mindestens $2 \cdot 10^{-5}$ Pa verlangt.

Außerdem wurde der 35 g/m² schwere Basisvliesstoff auch
ohne CRUSHED FOAM-Beschichtung wie oben beschrieben hydrophob ausgerüstet, um feststellen zu können, welchen Anteil
die Basis zur Verbesserung der Wasserdichtheit beiträgt.
Die Wasserdichtheit betrug in diesem Fall $1,1 \cdot 10^{-5}$ Pa.
Dies bedeutet, daß der gepreßte Schaum auch nach der Ausrüstung nur $6 \cdot 10^{-6}$ Pa zur Gesamtwasserdichtheit von
$1,7 \cdot 10^{-5}$ Pa beiträgt. In Anbetracht des relativ hohen
Auflagegewichtes von 30 g/m² ist dieser Wert sehr schlecht.

Beispiel 2

Das 35 g/m² schwere Basismaterial aus Beispiel 1 wurde
mit 30 g/m² (fest) einer Paste folgender Rezeptur beschichtet:

13

| Rezepturbestandteile | Teile fest | Teile flüssig |
|---|---|---|
| Wasser kalt | - | 26,26 |
| Pigmentfarbstoff | 0,315 | 0,63 |
| anionisches Schaumhilfsmittel | 0,800 | 4,00 |
| flüssiges, fettsäuremodifiziertes, schwach kationisches Melaminharz; 40%ige Emulsion | 12,000 | 30,00 |
| Methylcellulose; 0,3 %ig [+)] | 0,300 | 10,00 |
| Ammoniumstearat; 50%ig | 11,000 | 22,00 |
| Polyacrylsäureester-Dispersion; 50%ig | 20,000 | 40,00 |
| emulgatorarme Dispersion eines weichen Polyacrylsäureesters; 45%ig | 80,000 | 178,00 |
| Summe | 124,315 | 310,79 |

Feststoffgehalt der Mischung: 40 %

[+)] Hochmolekulares Granulat mit mittlerer Verätherung, Substitutionsgrad 1,4 - 1,6. Die 2%ige Lösung hatte eine Viskosität von 20 Pa s, gemessen mit einem Haake-Rotationsviskosimeter bei 20 °C und einem Schergefälle von 2,72 s$^{-1}$ (DIN 53 788).

14

Die 40%ige Mischung hatte ungeschäumt eine Viskosität
nach Brookfield (DIN 53 019) von 2,5 Pa s, gemessen
bei 20 °C mit Spindel Nr. 4 und 20 U/min. Sie wurde in
einem Schüttelmischer auf ein Schaumtopfgewicht von
0,13 g/m$^3$ gebracht. Die Konsistenz dieses extrem leichten
Schaumes war, ähnlich der eines Rasierschaumes, sehr feinporig und stabil. Die Brookfield-Viskosität des Schaumes
betrug 7,5 Pa s, gemessen mit Spindel Nr. 3 bei 20 U/min.

Mittels eines Streichwerkes, bestehend aus einer Stahlwalze
und einem Rakelmesser, wurden 32,5 g/m$^2$ fest auf den
Basisvliesstoff aufgetragen und in einem Bandtrockner bei
130 °C schonend getrocknet, um ein Aufreißen der Beschichtungsoberfläche aufgrund zu schneller Verdampfung
des Wassers zu verhindern. Der Schaum kollabierte während
der Trocknung nicht, sondern behielt sein Volumen bei.
Anschließend wurde der Schaum zwischen zwei kalten Stahlwalzen mit einem Liniendruck von 98 N/cm zusammengepreßt.
Die Wasserdichtheiten, gemessen an 6 unterschiedlichen
Stellen, lagen zwischen 3,2 . $10^{-5}$ Pa (erster Wassertropfen) und 4,2 . $10^{-5}$ Pa (dritter Tropfen), gemessen
nach DIN 53 886-77. Das Produkt hatte ein Durchschnittsgewicht von 67,4 g/m$^2$, davon 32,4 g/m$^2$ Schaumbeschichtung.

Weitere Prüfdaten:
Luftdurchlässigkeit bei 5 . $10^{-7}$ Pa (DIN 53 887):
140 dm$^3$/s m$^2$
Wasserdampfdurchlässigkeit, gemessen nach Mitton (DIN
53 333): 12,4 mg/cm$^2$ h
Nadelausreißkraft:   längs        4,5 N;
                     quer         6,3 N

Fallkoeffizient als Maß der Drapierfähigkeit (nach DIN 54 306):  58,7 %

Linting:  kein Partikelabtrag, abriebbeständig auf der Schaumseite.

Bereits ohne nachträgliche Hydrophobierung wird ein extrem hoher Wasserdichtheitswert erreicht. Die Ware ist gut wasserdampf- und luftdurchlässig und besitzt außerdem einen extrem niedrigen Fallkoeffizienten. Die nach Beispiel 2 hergestellte Ware ist somit allen nach dem Stand der Technik beschichteten und unbeschichteten Einweg-OP-Abdecktuch- und Kittelmaterialien weit überlegen.

Beispiel 3
‾‾‾‾‾‾‾‾‾‾

Das wie in Beispiel 2 hergestellte, beschichtete Material wurde zusätzlich gewaschen, naß-in-naß mit einer Hydrophobiermittelemulsion imprägniert und bei 145 °C auf einem Zylindertrockner getrocknet. Die Hydrophobiermittelflotte bestand aus einer zirkoniumsalzhaltigen, wäßrigen Paraffinemulsion mit einem Festgehalt von 8 %. Die Naßaufnahme, bezogen auf nasse Ware, betrug 25 % und bezogen auf lufttrockene Ware 35 %. Das Fertigmaterial wog durchschnittlich 69,3 g/m². Diese nachträgliche Ausrüstung steigerte die Wasserdichtheit von $3,8 \cdot 10^{-5}$ Pa auf $4,6 \cdot 10^{-5}$ Pa, und außerdem konnte die schwache Oberflächenklebrigkeit von Beispiel 2 völlig abgebaut werden.

Beispiel 4
——————————

Die in Beispiel 2 eingesetzte hochmolekulare Methylcellulose wurde durch gleiche Festteile einer mittelverätherten, niedrigmolekularen Type (Haake-Viskosität (DIN 53 788)
von 3 Pa s in einer 2%igen Lösung) ersetzt. Die Brookfield-Viskosität (DIN 53 019) der 40%igen ungeschäumten
Paste war mit 3,2 Pa s (Spindel Nr. 4, 20 U/min) höher
als die des Beispiels 2. Nach dem Aufschäumen auf ein
Topfgewicht von 0,13 g/cm³ wurde eine Brookfield-Viskosität
von 7,5 Pa s (Spindel Nr. 3, 20 U/min) gemessen. Die
Schaumpaste wurde wie in Beispiel 2 appliziert. Die Wasserdichtheiten, gemessen an 4 Proben, schwankten sehr wenig,
und zwar von mindestens $4,6 \cdot 10^{-5}$ bis maximal $5,15 \cdot 10^{-5}$ Pa.
Die arithmetisch gemittelte Wasserhaltefähigkeit betrug
$4,8 \cdot 10^{-5}$ Pa (DIN 53 886-77) ohne nachträgliche
Hydrophobierung. Das durchschnittliche Fertigmaterialgewicht
belief sich auf 68,4 g/m², davon 33,4 g/m² Schaum.

Beispiel 5
——————————

Im Vergleich zu Beispiel 4 wurde der Anteil an Ammoniumstearat um 50 % verringert und die gesamte Menge an
fettsäuremodifiziertem, leicht kationischem Harz durch
2,5 Teile (fest) einer kationenaktiven Emulsion eines
polymeren fluorierten Kohlenwasserstoffes ersetzt. Hier
wurde also ein Hydrophobiermittel verwendet, welches
gleichzeitig eine Antiblockwirkung auf die Beschichtung
ausübt.

Der Feststoffgehalt der Schaummischung wurde wieder auf
40 % eingestellt. Die Viskosität, gemessen nach Brookfield
(DIN 53 019) bei 20 U/min mit Spindel Nr. 4 betrug im
ungeschäumten Zustand 1,35 Pa s und nach dem Aufschäumen
auf 0,13 g/cm³ 7,52 Pa s (Spindel Nr. 3, 20 U/min).
Nach der Abarbeitung wie in Beispiel 2 wurde mit einem
durchschnittlichen Fertigmaterialgewicht von 67,4 g/m²
eine Wasserdichtheit von 4,2 . $10^{-5}$ Pa nach DIN 53 886-77
und eine völlig blockfreie Beschichtungsoberfläche erreicht.

Ein nach Beispiel 5 gefertigtes Material kann genauso
wie alle vorherigen Muster sowohl als Einweg-OP-Kittel- als
auch -Abdeckmaterial eingesetzt werden.

Beispiel 6

Ein mit Querkrempeln abgelegter, zweischichtiger Stapelfaserflor aus 15 g/m² eines Faserverschnittes, bestehend
aus 95 % Perlon und 5 % Zellwolle, und aus 15 g/m² eines
Verschnittes aus 25 % Perlon und 75 % Zellwolle wurde
unter den gleichen Bedingungen wie bei Beispiel 1
thermisch verfestigt. Die Verschweißungsfläche betrug
jedoch 14 % anstatt 24 % und die Rastertiefe 0,65 mm.
Aufgrund des speziellen Aufbaues und der größeren Rastertiefe wurde ein im Vergleich zu Beispiel 2 einseitig
extrem flusiges, voluminöses und sehr weiches Vlies
erzeugt. Die flusigere der beiden Seiten wurde, wie
unter Beispiel 2 beschrieben, mit 29 g/m² Schaum abgedeckt.

Anschließend wurde gewaschen und naß-in-naß mit einer 8%igen Paraffinemulsion nachgerüstet. Es wurde eine Wasserdichtheit von 3 . $10^{-5}$ Pa erzielt (DIN 53 886-77) bei einem durchschnittlichen Fertigmaterialgewicht von 61 g/m².

Luftdurchlässigkeit bei 5 . $10^{-7}$ Pa: 120 dm³/s m² (DIN 53 887)

Luftdurchlässigkeit bei 2 . $10^{-6}$ Pa: 450 dm³/s m²

Wasserdampfdurchlässigkeit nach Mitton (DIN 53 333): 33,2 mg/cm² h

Fallkoeffizient nach DIN 54 306:

    Schaumseite in der Meßanordnung nach oben:  35,4 %

    Schaumseite in der Meßanordnung nach unten: 32,3 %.

Mit keiner der bisher bekannten Methoden zur Beschichtung oder Wasserdichtausrüstung kann ein dermaßen exzellentes Fallvermögen bei hoher Wasserdichtheit einerseits und hoher Luftdurchlässigkeit und Wasserdampfdurchlässigkeit andererseits erzielt werden.

Patentansprüche:

1. Wegwerfbares Abdeck- oder Bekleidungstuch für die Chirurgie, welches einen drapierfähigen Trägervliesstoff und eine darauf befindliche, diesen nicht durchdringende, polymere, wasserdichte Deckschicht aufweist, dadurch gekennzeichnet, daß die Deckschicht aus einem drapierfähigen, wasserdampfdurchlässigen, emulgatorarmen, verpreßten Polymerschaumfilm auf der Basis Polyacrylsäureester, Polyvinylacetatacrylat und/oder Nitrilbutadien mit einem Flächengewicht von 15 bis 50 g/m² besteht, der - jeweils bezogen auf das Schaumgewicht -
1 bis 15 % Hydrophobiermittel, 3 bis 15 % eines Ammonium- oder Alkylammoniumsalzes einer langkettigen Fettsäure mit einer Kohlenstoffzahl zwischen 14 und 20 als Schaumstabilisator sowie mindestens 0,25 % Methyl-oder Methylhydroxyalkylcellulose als Verdickungsmittel enthält.

2. Abdeck- oder Bekleidungstuch nach Anspruch 1, dadurch gekennzeichnet, daß der Schaumstabilisator Ammoniumstearat ist.

3. Abdeck- oder Bekleidungstuch nach Anspruch 1 oder 2, dadurch gekennzeichnet, daß der Schaum als Hydrophobiermittel
fettsäuremodifizierte Melaminharze,
deren Mischungen mit Paraffin,
zirkoniumsalzhaltige, wäßrige Paraffinemulsionen,
polymere fluorierte Kohlenwasserstoffe oder
deren Mischungen mit den genannten Substanzen enthält.

4.  Abdeck- oder Bekleidungstuch nach einem oder mehreren der vorhergehenden Ansprüche, dadurch gekennzeichnet, daß die Vliesstoffträgerschicht hydrophob ist.

5.  Abdeck- oder Bekleidungstuch nach einem oder mehreren der vorhergehenden Ansprüche, dadurch gekennzeichnet, daß die Beschichtung Antiblockmittel enthält.

6.  Verfahren zur Herstellung eines wegwerfbaren Abdeck- oder Bekleidungstuches für die Chirurgie, bei dem mittels der CRUSHED FOAM-Technik auf einen drapierfähigen Trägervliesstoff einseitig mittels Rakeln und/oder Walzen eine wäßrige Polymerschaumdispersion aufgetragen wird, dadurch gekennzeichnet, daß die Polymerschaumdispersion auf der Basis Polyacrylester, Polyvinylacetatacrylat oder Nitrilbutadien besteht und emulgatorarm ist, wobei ihr Schaumgewicht weniger als 0,2 g/cm³ und ihr Auftragungsgewicht 15 bis 50 g/m² beträgt, und daß der Schaum - jeweils bezogen auf sein Gesamtgewicht -
1 bis 15 % Hydrophobiermittel,
3 bis 15 % eines Schaumstabilisators, bestehend aus einem Ammonium- oder Alklyammoniumsalz einer langkettigen Fettsäure mit einer Kohlenstoffzahl zwischen 14 und 20,
und mindestens 0,25 % Methyl- oder Methylhydroxyalkylcellulose als Verdicker
enthält.

7. Verfahren nach Anspruch 6, dadurch gekennzeichnet, daß die Viskositäten der ungeschäumten Mischung, gemessen nach DIN 53 019, 1 bis 2 Pa s betragen.

8. Verfahren nach Anspruch 6 oder 7, dadurch gekennzeichnet, daß als Schaumstabilisator Ammoniumstearat verwendet wird.

9. Verfahren nach einem oder mehreren der vorhergehenden Ansprüche, dadurch gekennzeichnet, daß die Methyl- oder Methylhydroxyalkylcellulose mittel- bis hochveräthert ist.

10. Verfahren nach einem oder mehreren der vorhergehenden Ansprüche, dadurch gekennzeichnet, daß als Hydrophobiermittel
fettsäuremodifizierte Melaminharze,
deren Mischungen mit Paraffin,
zirkoniumsalzhaltige, wäßrige Paraffinemulsionen,
polymere fluorierte Kohlenwasserstoffe oder
deren Mischungen mit den genannten Substanzen verwendet werden.

11. Verfahren nach einem oder mehreren der vorhergehenden Ansprüche, dadurch gekennzeichnet, daß das Produkt nach der Herstellung zusätzlich gewaschen, dann der Trägervliesstoff direkt anschließend mit einer Hydrophobierungsmittelemulsion imprägniert und der Schichtstoff sodann getrocknet wird.

12. Verfahren nach einem oder mehreren der vorhergehenden Ansprüche, dadurch gekennzeichnet, daß die
fertige Beschichtung mit einem Antiblockmittel getränkt oder der Schaum vor dem Zusammenpressen mit
einem solchen bepudert wird.